Europäisches Patentamt

European Patent Office

Office européen des brevets

(19)

(11) Veröffentlichungsnummer: **0 266 631 B1**

(12) # EUROPÄISCHE PATENTSCHRIFT

(45) Veröffentlichungstag der Patentschrift: **18.09.91**

(51) Int. Cl.5: **G01N 33/44, G01N 3/00**

(21) Anmeldenummer: **87115392.0**

(22) Anmeldetag: **21.10.87**

Die Akte enthält technische Angaben, die nach dem Eingang der Anmeldung eingereicht wurden und die nicht in dieser Patentschrift enthalten sind.

(54) **Prüfvorrichtung zur Feststellung mechanischer Eigenschaften von Granulat.**

(30) Priorität: **03.11.86 DE 8629187 U**

(43) Veröffentlichungstag der Anmeldung:
**11.05.88 Patentblatt 88/19**

(45) Bekanntmachung des Hinweises auf die
Patenterteilung:
**18.09.91 Patentblatt 91/38**

(84) Benannte Vertragsstaaten:
**BE DE ES FR GB IT**

(56) Entgegenhaltungen:
**EP-A- 0 021 083**
**FR-A- 2 335 834**
**US-A- 2 572 779**
**US-A- 3 014 365**
**US-A- 3 485 089**

(73) Patentinhaber: **BAYER AG**

**W-5090 Leverkusen 1 Bayerwerk(DE)**

(72) Erfinder: **Walzel, Peter, Dr.**
**Von-Ketteler Strasse 4**
**W-4047 Dormagen(DE)**
Erfinder: **Juffa, Richard**
**Buchholzstrasse 38**
**W-5000 Koeln 80(DE)**

EP 0 266 631 B1

## Beschreibung

Die Erfindung betrifft eine Prüfvorrichtung zur Feststellung mechanischer Eigenschaften - insbesondere der Zugfestigkeit - bei der Einmischung von Granulat in unvulkanisierten Kautschuk, bestehend aus einer Unterstützung für den Prüfkörper, einem Keil und einem linear wirkenden Druckerzeuger.

In einen Rohkautschuk werden vor der Vulkanisation verschiedene Zusatzstoffe eingemischt. Dadurch kann das Vulkanisationsverhalten gesteuert und die physikalischen Eigenschaften des fertigen Gummiteils und dessen Alterungsbeständigkeit verbessert werden.

Früher wurden dieses Zusatzstoffe ausschließlich in Form von feinkörnigen Pulvern dem Kautschuk beigefügt. Aus Gründen der Arbeitshygiene, und einer besseren Dosierbarkeit setzt man seit einiger Zeit in verstärktem Maß agglomerierte Zusatzstoffe (Granulate) ein.

Je nach Stoffaufbau und Verarbeitung weisen diese unterschiedliche Eigenschaften hinsichtlich Porosität, Härte, Zugfestigkeit und Dispergierverhalten während des Einarbeitens in den Kautschuk auf. Für die Qualität der fertigen Gummiteile, insbesondere von technischen Artikeln, wie zum Beispiel Autoreifen, ist es aber von ausschlaggebender Bedeutung, daß das Granulat während der Einarbeitung wieder möglichst fein zerfällt, um eine optimale Ausnutzung im Kautschuk zu erzielen und Zerstörung des Gummis bei Lasteinwirkung infolge von Anrissen durch zu große Teilchen zu vermeiden. Es werden also zwei konträre Eigenschaften gefordert. Zur Handhabung wird ein festes Agglomerat benötigt, welches dann während der Einarbeitung in den Roh-Kautschuk leicht und vollständig wieder in die das Granulat aufbauenden Körner zerfällt.

Es ist bekannt, in einem Laborwalzwerk Granulate in einen Kautschuk einzumischen und anschließend durch Sichtproben festzustellen, ob das Granulat wieder fein zerfallen ist.

Die Prüfung ist aber sehr ungenau, da es sehr schwierig ist, die Feinstanteile noch einwandfrei feststellen zu können. Auch ist die Prüfung sehr aufwendig, so daß sie für die Produktionskontrolle nicht besonders geeignet ist.

Aus US-3 014 365 ist ein Teilchenprüfgerät bekannt, mit dem durch einen am Ende kugelförmig ausgebildeten Stab meßbarer Druck auf die nicht unterstützte Mitte des Teilchens ausgeübt wird.

Aufgabe der Erfindung ist es, eine Prüfvorrichtung zu finden, die bereits vor dem Einmischen des agglomerierten Zusatzstoffes (Granulat) in einen unvulkanisierten Kautschuk eine ausreichende Aussage über Verarbeitungseigenschaften anhand von Parametern (Scherkraft, Zugfestigkeit) in kurzer Zeit (10-20 min) erlaubt.

Die Aufgabe wird erfindungsgemäß dadurch gelöst, daß als Unterstützung für den Prüfkörper linienförmige Auflagen quer zur Wirkungsebene des verschiebbaren flachen Keils angeordnet sind.

Es ist überraschend, daß durch Spaltung der kleinen Granulate hinreichend genaue Parameter zur Beurteilung der Dispergierfähigkeit beim Zugeben dieser Zusatzstoffe zum Kautschuk ermittelt werden können.

Der Keil bildet nämlich beim Aufsetzen auf das Agglomerat, insbesondere Granulat, einen im Querschnitt dreieckförmigen Stoffkeil im oberen Berührungsbereich aus, der bei weiterer Belastung den darunterliegenden Querschnitt auseinander sprengt, wodurch senkrecht zur späteren Spaltfläche Zugkräfte entstehen. Der Kraftwegeverlauf wird während dieses Vorganges aufgezeichnet. Hieraus lassen sich Schlüsse über die Einarbeitbarkeit des Zusatzstoffes im Kautschuk ziehen, wenn dieser später als Granulat im Kalander eingesetzt wird.

Die Spaltung eines einzelnen Granulates benötigt nur sehr kurze Zeit, so daß eine große Anzahl von Proben in einer annehmbaren Zeit 10-30 min geprüft werden kann, so daß auch bei nicht exakten Formen, wie sie nun einmal beim Herstellen eines Granulates vorliegen, über einen statistischen Mittelwert eine Aussage über die Einarbeitbarkeit gemacht werden kann.

Bei dem üblicherweise verwendeten zylindrischen Granulat berührt die gerade Schneide des Keils anfangs dieses nur in der Mitte, um dann den Stoffkeil von innen nach außen aufzubauen, wodurch vorzeitige seitliche Ausbrüche vermieden werden. Weiter behindert die untere linienförmige Auflage quer zur Wirkungsebene des Keils nicht die Spaltung, da das Granulat in Richtung der Längsachse sich frei bewegen kann, so daß sich ein unterer störender Stoffkeil kaum ausbildet. Dieser Vorgang wird noch unterstützt, wenn beispielsweise auf jeder Seite der senkrechten Mittelebene des liegenden zylindrischen Granulats je eine linienartige Auflage angeordnet ist.

Vorteilhaft ist dabei eine Zufuhreinrichtung, die aus einem linear verschiebbaren oder drehbaren Magazin bestehen kann, welches gleichzeitig die Granulate ausrichtet und als Auflage während der Spaltung dient oder nur die Granulate der zur Spaltung dienenden Auflage zufügt.

Durch die Anordnung eines entgegengesetzt in der Wirkungsebene des Keils verschiebbaren Konterkeils ist es möglich, die Probe symmetrisch zu beaufschlagen, so daß die beidseitigen nahezu symmetrischen Schubzonen in den Zugbereich übergehen.

Es hat sich als vorteilhaft erwiesen, den Keil vorne abzurunden, um auch bei weicherem Granulat einen entsprechend großen Stoffkeil ausbilden zu können. Zweckmäßigerweise werden bei den Versuchen mit zylindrischen, stranggepreßten Kautschuk-Zusatzstoffen Granulate mit Durchmesser D von 1-4 mm, insbesondere 1,5-2,5 mm, verwendet. Die Radien R der vorderen Abrundung des Keiles betragen 0,1-0,4 mm. Der Quotient R/D sollte zwischen 0,05 und 0,2, insbesondere zwischen 0,075 und 0,125, liegen.

Durch ein Gelenk zwischen beispielsweise einem Keil und einer Schubstange des Hydraulikzylinders als Druckerzeuger erfolgt eine selbsttätige Zentrierung, so daß auch bei kleinen Unebenheiten ein gleichartiger Stoffkeil aufgebaut wird, wodurch die Meßgenauigkeit erhöht wird.

Schließlich kann durch ein elastisches, gedämpftes Zwischenglied zwischen Druckerzeuger und Keil ein hartes Aufsetzen auf das Granulat vermieden werden. Das Material hat also genügend Zeit, den Stoffkeil auszubilden, bevor der eigentliche Spaltvorgang einsetzt.

Die linienartigen abstützenden Auflagen erstreckten sich quer- und beiderseits der Wirkungsebene des Keils, wobei sich rechteckige bzw. V-förmige Querschnitte oder andere polygonartige Begrenzungen als besonders günstig erwiesen haben.

Wenn die Vorschubgeschwindigkeit stetig oder in Stufen geändert werden kann, ist es möglich, die Nebenzeiten (z.B. Rücklauf) zu reduzieren. Wichtig ist andererseits, dem Material aufgrund seiner Trägheit ausreichend Zeit zu geben, um den Stoffkeil sauber ausbilden zu können, danach kann die weitere Prüfung der Zugspannung mit großer Vorschubgeschwindigkeit erfolgen.

Als Prüfkörper sind alle Granulate mit zylindrischem, elliptischem, mehreckigem und kugelförmigen Querschnitt geeignet.

Ein Beispiel der Prüfvorrichtung ist in den Zeichnungen dargestellt und wird im folgenden erläutert. Es zeigen

Fig. 1    Seitenansicht der Prüfvorrichtung
Fig. 2    Teller für Granulat
Fig. 3    Kurvendiagramm Bruchkraft/Tiefe
Fig. 4    Histogramm mit geringer Streuung
Fig. 5    Histogramm mit breiter Streuung

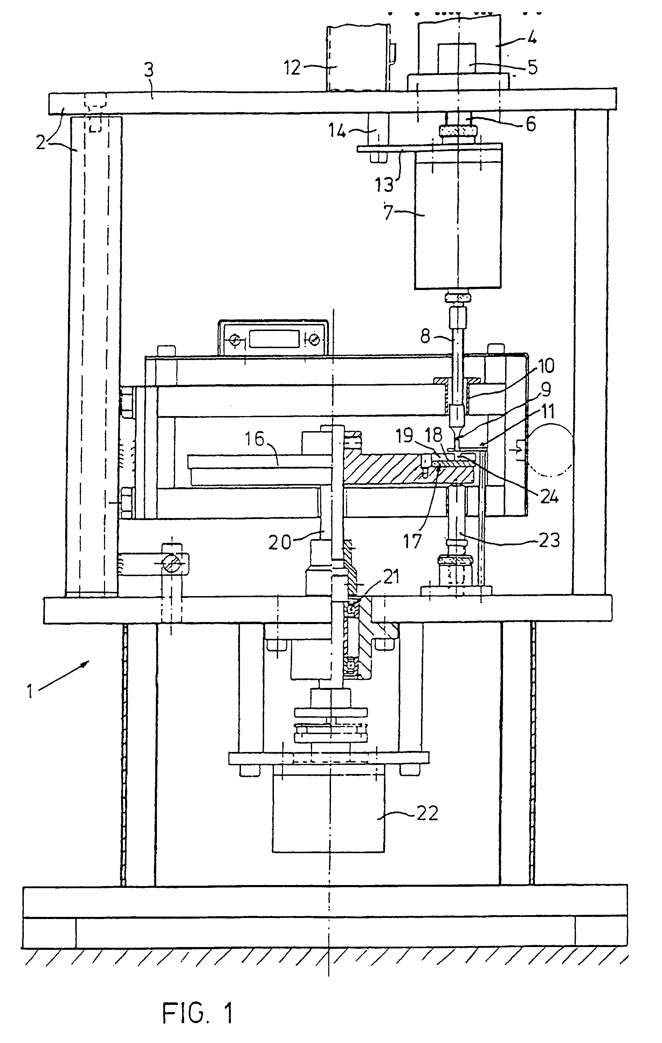

In Fig. 1 ist eine Prüfvorrichtung 1 dargestellt, bei der ein aufgeständerter Rahmen 2 die einzelnen Bauteile trägt.

Über dem oberen Riegel 3 ist ein Druckerzeuger 4 angeordnet, der aus einem regelbaren Motor 5 und einer Spindel 6 besteht, die wiederum über einen Kraftaufnehmer 7 und eine Schubstange 8 mit dem austauschbaren Keil 9 verbunden ist. Dieser Keil 9 wird im oberen Bereich durch ein Gleitlager 10 geführt und ist im unteren Bereich von einem Abstreifer 11 umgeben.

Weiter ist am oberen Riegel 3 ein Potentiometer 12 angeordnet, welches mit dem Kraftaufnehmer 7 über einen Hebel 13 und einer Stange 14 verbunden ist.

Auf dem unteren Riegel 15 ist als Zufuhreinrichtung 16 ein Drehtisch angebracht, an dessen äußeren Kreisring 17 sich radial ausgerichtete V-förmige Rinnen 18 mit parallelen Auflagen 19 befinden. Der Drehtisch 16 ist um die Mittelachse 20 in Kugellagern 21 durch Zweiphasen-Motor 22 schrittweise drehbar.

Ferner kann im Bereich des Kreisringes 17 eine Abstützung durch Hubstempel 23 erfolgen.

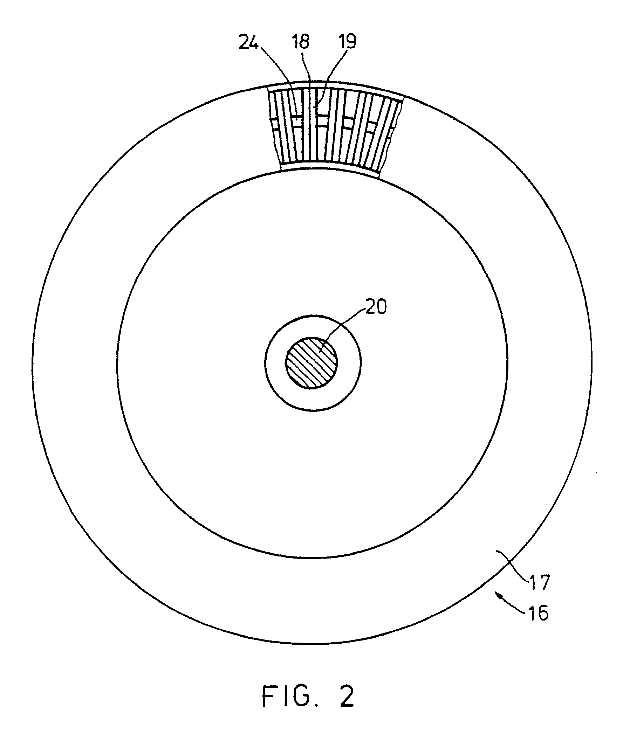

In Fig. 2 ist ein Drehtisch 16 gezeichnet, bei dem auf einen äußeren Kreisring 17 rechteckförmige Rinnen 18 mit Bodenfläche und parallelen senkrechten Wänden 19 zur Fixierung des zylindrischen Granulats angeordnet sind, wobei in der Rinnenmitte ein freier, in Umfangsrichtung verlaufender Spalt 24 zum mindestens teilweisen Eintauchen des Keiles 9 vorgesehen ist.

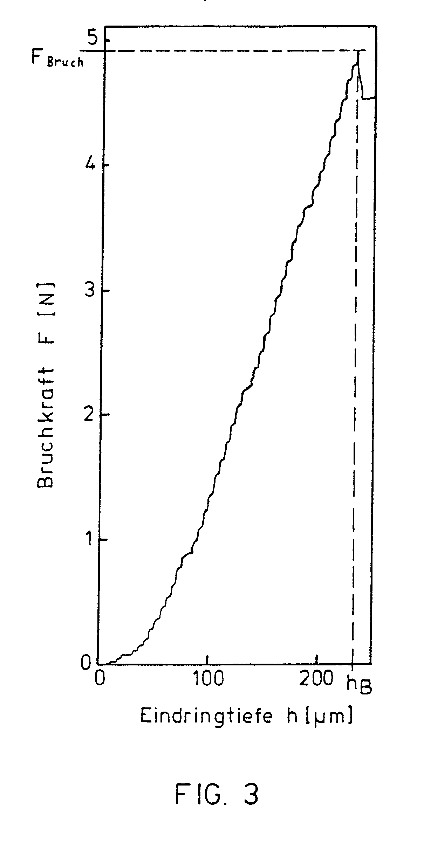

In Fig. 3 ist ein Diagramm über den Kraft-/Wegverlauf dargestellt. Bei geringem Kraftanstieg verläuft die Kurve erst zur Ausbildung des Stoffkeils sehr flach, um dann fast proportional bis zur Bruchlast anzusteigen.

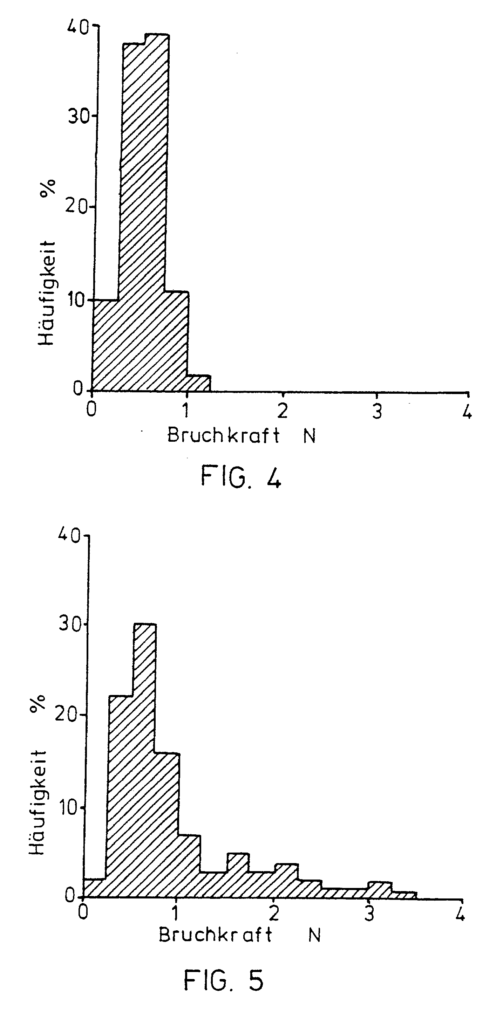

Fig. 4 und 5 zeigen Histogramme der gemessenen Bruchkräftevon jeweils 100 zylindrischen Granulaten, die durch Rollgranulieren bzw. durch Strangpressen agglomeriert wurden. Fig. 4 weist eine enge Häufigkeitsverteilung bei geringen maximalen Bruchkräften auf, deshalb wird dieses Granulat beim Einmischen gut dispergieren, während Fig. 5 eine breitgestreute Häufigkeitsverteilung mit großem Mittelwert der Bruchkraft aufweist. Dieses Prüfungsergebnis läßt auf eine schlechte Einarbeitbarkeit des Granulats in den Kautschuk schließen.

Das Verfahren lief dabei in folgenden Schritten ab.

Der Stempel wird bis auf eine Entfernung von 0,5 mm im Schnellauf (1 mm/min) auf das Granulat abgesenkt. Anschließend wird der Stempel mit konstanter Geschwindigkeit langsam weiter abgesenkt bis sich der Stoff-Keil im Granulat ausbildet und das Granulat bricht. Der dabei auftretende Maximalwert der Kraft wird gespeichert. Die Absenkgeschwindigkeit des Keils liegt im Bereich von 1-10 mm/min. Aus der aufgezeichneten Kraftanstiegskurve kann auf die Struktur der Granulate und deren Dispergierbarkeit in den Kautschuk geschlossen werden. Nach dem Bruch wird der Stempel rasch hochgefahren. Das fallweise am Keil klebende Granulat wird von einem Abstreifer am Hochgehen gehindert und fällt auf den Teller zurück. Nach dem Positionieren des nächsten Granulates durch Weiterdrehen des Tellers kann die nächste Messung erfolgen.

**Patentansprüche**

1. Prüfvorrichtung zur Feststellung mechanischer Eigenschaften - insbesondere Zugfestigkeit - bei der Einmischung von Granulat in unvulkanisierten Kautschuk, bestehend aus einer Unterstützung für den Prüfkörper, einem Keil und einem linear wirkenden Druckerzeuger, dadurch gekennzeichnet, daß als Unterstützung für den Prüfkörper linienförmige Auflagen (19) quer zur Wirkungsebene des verschiebbaren flachen Keils (9) angeordnet sind.

2. Prüfvorrichtung nach Anspruch 1, dadurch gekennzeichnet, daß eine Zufuhrvorrichtung (16) für die Granulate vorhanden ist.

3. Prüfvorrichtung nach Anspruch 2, dadurch gekennzeichnet, daß als Zufuhrvorrichtung (16) ein drehbares, horizontales Magazin mit einzelnen radial ausgerichteten Rinnen (18) angeordnet ist.

4. Prüfvorrichtung nach Anspruch 1-3, dadurch gekennzeichnet, daß auf der unteren Seite ein hebbarer Konterkeil vorhanden ist.

5. Prüfvorrichtung nach Ansprüchen 1-4, dadurch gekennzeichnet, daß der Keil (9) vorne abgerundet ist und die vordere Abrundung einen Radius von 0,2-0,4 mm, insbesondere von 0,15-0,25 mm, besitzt.

6. Prüfvorrichtung nach Anspruch 1-5, dadurch gekennzeichnet, daß der Keil (9) gelenkig aufgehängt ist.

7. Prüfvorrichtung nach Ansprüchen 1-6, dadurch gekennzeichnet, daß zwischen Druckerzeuger (4) und Keil (9) ein elastisches und wärmeisolierendes Zwischenglied eingebaut ist.

8. Prüfvorrichtung nach Ansprüchen 1-7, dadurch gekennzeichnet, daß der Druckerzeuger (4) eine veränderbare Vorschubgeschwindigkeit besitzt.

**Claims**

1. Test apparatus for determining mechanical properties -in particular tensile strength - when incorporating granular material into unvulcanised rubber, comprising a support for the test piece, a wedge and a linearly operating pressure generator, characterised in that, as a support for the test piece, linear rests (19) are arranged transversely relative to the plane of action of the displaceable flat wedge (9).

2. Test apparatus according to claim 1, characterised in that a feed device (16) for the granular materials is provided.

3. Test apparatus according to claim 2, characterised in that a rotatable horizontal magazine having individual radially aligned channels (18) is disposed as a feed device (16).

4. Test apparatus according to claims 1-3, characterised in that a raisable counter wedge is provided on the bottom side.

5. Test apparatus according to claims 1-4, characterised in that the wedge (9) is rounded off at the front and the front rounding-off has a radius of 0.2 - 0.4 mm, in particular of 0.15 - 0.25 mm.

6. Test apparatus according to claims 1-5, characterised in that the wedge (9) is suspended in an articulated manner.

7. Test apparatus according to claims 1-6, characterised in that an elastic and thermally insulating intermediate member is fitted between pressure generator (4) and wedge (9).

8. Test apparatus according to claims 1-7, characterised in that the pressure generator (4) has a variable feed rate.

**Revendications**

1. Dispositif d'essai pour la détermination des propriétés mécaniques - en particulier la résistance à la traction - pour l'incorporation de granulats dans du caoutchouc non vulcanisé, composé d'un support pour l'éprouvette, d'un coin et d'un générateur de pression à action linéaire, caractérisé en ce que le support pour l'éprouvette consiste en appuis linéaires (19) perpendiculaires au plan de travail du coin plat coulissant (9).

2. Dispositif d'essai selon la revendication 1, caractérisé en ce qu'un dispositif d'amenée (16) est prévu pour les granulats.

3. Dispositif d'essai selon la revendication 2, caractérisé en ce que le dispositif d'amenée (16) consiste en un magasin rotatif horizontal avec des rainures (18) alignées en direction radiale.

4. Dispositif d'essai selon les revendications 1 à 3, caractérisé en ce qu'un contre-coin levable est prévu à la partie inférieure.

5. Dispositif d'essai selon les revendications 1 à 4, caractérisé en ce que le coin (9) est arrondi à son extrémité d'attaque et que l'arrondi possède un rayon de 0,2 à 0,4 mm, en particulier de 0,15 à 0,25 mm.

6. Dispositif selon les revendications 1 à 5, caractérisé en ce que le coin (9) est suspendu de manière articulée.

7. Dispositif d'essai selon les revendications 1 à 6, caractérisé en ce qu'un élément intermédiaire élastique et isolant thermique est introduit entre le générateur de pression (4) et le coin (9).

8. Dispositif d'essai selon les revendications 1 à 7, caractérisé en ce que le générateur de pression (4) possède une vitesse d'avance variable.

FIG. 1

FIG. 2

FIG. 3

FIG. 4

FIG. 5